(19) Europäisches Patentamt
European Patent Office
Office européen des brevets

(11) EP 1 300 397 A1

(12) EUROPÄISCHE PATENTANMELDUNG

(43) Veröffentlichungstag:
**09.04.2003 Patentblatt 2003/15**

(51) Int Cl.[7]: **C07D 233/64**, C07K 1/06

(21) Anmeldenummer: **02027593.9**

(22) Anmeldetag: **10.06.1994**

(84) Benannte Vertragsstaaten:
**CH DE FR GB IT LI NL SE**

(30) Priorität: **11.06.1993 DE 4319475**
**18.06.1993 DE 4320260**

(62) Dokumentnummer(n) der früheren Anmeldung(en) nach Art. 76 EPÜ:
**94920441.6 / 0 702 674**

(71) Anmelder: **Gesellschaft für Biotechnologische Forschung mbH (GBF)**
**D-38124 Braunschweig (DE)**

(72) Erfinder:
- **Frank, Ronald**
  **38124 Braunschweig (DE)**
- **Hoffmann, Stefan**
  **Braunschweig (DE)**

(74) Vertreter: **Boeters, Hans Dietrich, Dr. et al**
**Patentanwälte Boeters & Bauer,**
**Bereiteranger 15**
**81541 München (DE)**

Bemerkungen:
Diese Anmeldung ist am 10 - 12 - 2002 als Teilanmeldung zu der unter INID-Kode 62 erwähnten Anmeldung eingereicht worden.

# (54) Verbindung für Schutz- und Ankergruppen und deren Verwendung

(57) Die Erfindung betrifft Verbindungen, die mit einer Carboxyl-Funktion unter Ausbildung einer Esterbindung umgesetzt werden können, wodurch eine Schutzgruppe und insbesondere eine Ankergruppe für die Carboxyl-Funktion vorgesehen wird. Die Erfindung betrifft ferner Ester von Säuren, die durch Veresterung mit einer erfindungsgemäßen Verbindung gewinnbar sind, sowie die Verwendung der erfindungsgemäßen Verbindungen bei der Peptidsynthese.

A

Fig. 1

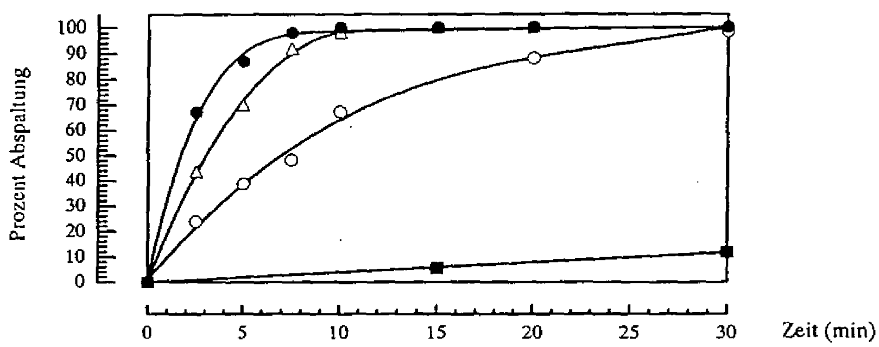


Kinetik der Peptidabspaltung von einigen Ankergruppen in Lösung. **A** : (•) Verbindung (9a) in 0.01 M $KH_2PO_4/NaHPO_4$ (pH 7.5), (▵) Verbindung (9a) in 0.01 M $KH_2PO_4/Na_2HPO_4$ (pH 7.5), (○) Verbindung (9a) in 0.01 M TEAAc (pH 7.3), (■) Verbindung (11a) in 0.01 M $KH_2PO_4/Na_2HPO_4$ (pH 7.5) bei 50°C; B : (•) Verbindung (9a), (○) Verbindung (8a) und (■) Verbindung (11a) in 0.01 M $KH_2PO_4$/ $Na_2HPO_4$ (pH 7.5) bei 25°C.



Printed by Jouve, 75001 PARIS (FR)

## Beschreibung

Die regioselektive chemische Umsetzung einer Verbindung mit mehreren auch unterschiedlichen reaktiven chemischen Funktionen erfordert den Schutz aller dieser Funktionen bis auf diejenige(n), mit der (denen) eine chemische Reaktion eingegangen werden soll. Zum Schutz dieser Funktionen werden Molekülgruppen (Schutzgruppen) eingeführt. Diese lassen sich im Anschluß schonend und selektiv unter Zurückbildung der ursprünglichen Funktion abspalten. Für komplexe und mehrstufige Synthesen, insbesondere von Naturstoffen, wie Oligopeptiden und Oligonucleotiden, sind verschiedene Typen von Schutzgruppen notwendig. Sie zeichnen sich durch stark unterschiedliche Bedingungen der Abspaltung aus. Ein System von Schutzgruppen, in dem die einzelnen Typen so selektiv spaltbar sind, daß jeweils alle anderen Schutzgruppen unberührt bleiben, wird *orthogonal* genannt. Der Fachmann ist mit dem Prinzip der Schutzgruppenchemie vertraut. Verwiesen sei auf die im folgenden angeführte Literatur. Weist der Schutzgruppentyp noch eine weitere reaktive Funktion auf, so daß er kovalent und stabil mit einem Trägermaterial für die Festphasensynthese verknüpft werden kann, dann spricht der Fachmann von einer "Anker/gruppe" oder "Linkergruppe". Ein spezieller Typ von Schutzgruppe liegt dann vor, wenn die Schutzgruppe erst durch eine der Abspaltung vorgeschaltete chemische Reaktion in eine labile Form gebracht werden muß, die dann in einem zweiten Schritt unter milden Bedingungen abgespalten werden kann. Man spricht in diesem Fall von einer geschützten Schutzgruppe beziehungsweise "safety-catch group". Obwohl hier zwei Reaktionsschritte zur Abspaltung notwending sind, kann eine derartige Gruppe doch große Vorteile bieten:

- sie kann sehr stabil gegen viele, auch sehr drastische Reaktionsbedingungen sein, jedoch durch die Folge von zwei spezifischen, sehr milden Reaktionsschritten abgespalten werden können;
- die labile Zwischenstufe der Schutzgruppe kann stabil genug sein, daß sie gute oder bessere Möglichkeiten zur Isolierung und Reinigung des Endproduktes bietet.

Aufgabe der vorliegenden Erfindung ist es, eine Verbindung vorzusehen, mit der sich eine Carboxyl-Funktion (COOH) unter Ausbildung einer Esterbindung umsetzen läßt, wodurch eine Schutzgruppe und insbesondere eine Ankergruppe für die Carboxyl-Funktion vorgesehen werden kann, die folgende Merkmale aufweist:

die Schutz- beziehungsweise Ankergruppe soll ihrerseits geschützt sein und sich in eine labile Zwischenstufe überführen lassen;
- die labile Zwischenstufe soll unter vorgegebenen Reaktionsbedingungen, beispielsweise denen einer Festphasensynthese von Peptiden, stabil sein und eine Reinigung der Zwischenprodukte erlauben;
- die labile Zwischenstufe soll in wässriger physiologischer Pufferlösung, vorzugsweise bei neutralem pH (etwa pH 7) oder nahezu neutralem pH (5 bis 9), zerfallen und die ursprüngliche Carboxyl-Funktion zurückbilden können, so daß das Syntheseprodukt mit freier Carboxyl-Funktion direkt (ohne weitere Reinigung) in ein zellbiologisches oder biochemisches Testexperiment einsetzbar ist.

Insbesondere soll dieser Schutzgruppentyp als Ankergruppe für die Festphasensynthese von Peptiden einsetzbar sein, insbesondere nach der Fmoc/tBu-Methode; vergleiche beispielsweise Fields & Noble in Int. J. Peptide Protein Res., 35 (1990) 161-214. Erfindungsgemäß wird dazu eine Verbindung der Formel

$$Y\text{-}C(R_2)(R_3)\text{-}B\text{-}X$$

vorgesehen, wobei die Verbindung mit einer Carboxyl-Funktion durch Substituieren der Y-Gruppe unter Ausbildung einer Esterbindung umgesetzt werden kann, wodurch eine Schutzgruppe und insbesondere eine Ankergruppe für die Carboxyl-Funktion vorgesehen wird, wobei

Y = HO, Cl, Br oder I,
B = basische Gruppe, die durch X geschützt ist, nur hydrolyseempfindliche Acylverbindungen eingeht und entschützt intramolekular die Hydrolyse der Esterbindung katalysieren kann,
X = eine Schutzgruppe für die basische Gruppe B bedeutet und deren Katalysevermögen blockiert,
$R_2$ und $R_3$ = beliebige Reste sind, die nicht die Bildung der Esterbindung und deren Hydrolyse beeinträchtigen.

Wie bereits gesagt, ist der Fachmann mit dem Prinzip der Schutzgruppenchemie vertraut. Zur Erläuterung sei auf folgenden Stand der Technik verwiesen.
Schutzgruppe (Protective Group): beispielsweise Greene & Wuts, Eds., Protective Groups in Organic Synthesis, 2nd Ed., 1991, John Wiley & Sons, NY.

Ankergruppe oder Linkergruppe: beispielsweise Breipohl et al. in Tetrahedron Lett., 28 (1987) 5651-5654; Guibe et al. in Tetrahedron Lett., 30 (1989) 2641-2644.
Geschützte Schutzgruppe (Safety-Catch Group): Patek in Int. J. Peptide Protein Res., 42 (1993) 97-117.
Elektronenziehende Schutzgruppe für eine basische Gruppe, wobei erstere die Basizität der basischen Gruppe herabsetzt: Gross & Meienhofer, Eds., The Peptides, Vol. 3, 1981, Academic Press, NY.; Patek in Int. J. Peptide Protein Res., 42 (1993) 97-117, insbesondere 112.

Die chemische Bindung zwischen Schutzgruppe und Carboxyl-Funktion soll also eine Esterbindung sein. Diese Esterbindung kann durch Umsetzung der Carboxyl-Funktion mit einer Hydroxyl-Funktion oder einer Halogen-Funktion der erfindungsgemäßen Verbindung eingeführt werden. Carbonsäureester sind in wässriger Lösung mehr oder weniger leicht durch Katalyse von Basen hydrolytisch spaltbar. Die Schutzgruppe besitzt daher eine basische Funktion B, welche intramolekular die Hydrolyse der Esterbindung katalysieren kann. Diese basische Funktion B darf selbst keine hydrolysestabilen Acylverbindungen eingehen, da sonst die zu schützende Carboxylverbindung auf B übertragen werden könnte und somit keine hydrolytische Spaltung eintritt. Diese basische Funktion ist darüberhinaus durch eine Gruppe X geschützt, die die Basizität der Gruppe B soweit herabsetzt, daß eine intramolekular katalysierte Hydrolyse der Esterbindung erst nach Abspaltung der Gruppe X erfolgen kann. Die Gruppe X ist unter den Bedingungen einer an $R_1$ ansetzenden Synthese stabil.

Das folgende Reaktionsschema soll die Verwendbarkeit der erfindungsgemäßen Verbindung näher erläutern. Dabei bedeuten:

$R_1$ = Rest der zu schützenden Verbindung,
$R_2$ und $R_3$ = Reste der Schutzgruppe, welche nicht an deren Funktion teilhaben (wenn $R_2$ oder $R_3$ eine zusätzliche reaktive Funktion für eine Verknüpfung mit Trägermaterialien aufweisen, beispielsweise -COOH, -OH, $-NH_2$ oder -SH, dann wird die Schutzgruppe als Ankergruppe eingesetzt),
B = basische Funktion und
X = Schutzgruppe der basischen Funktion.

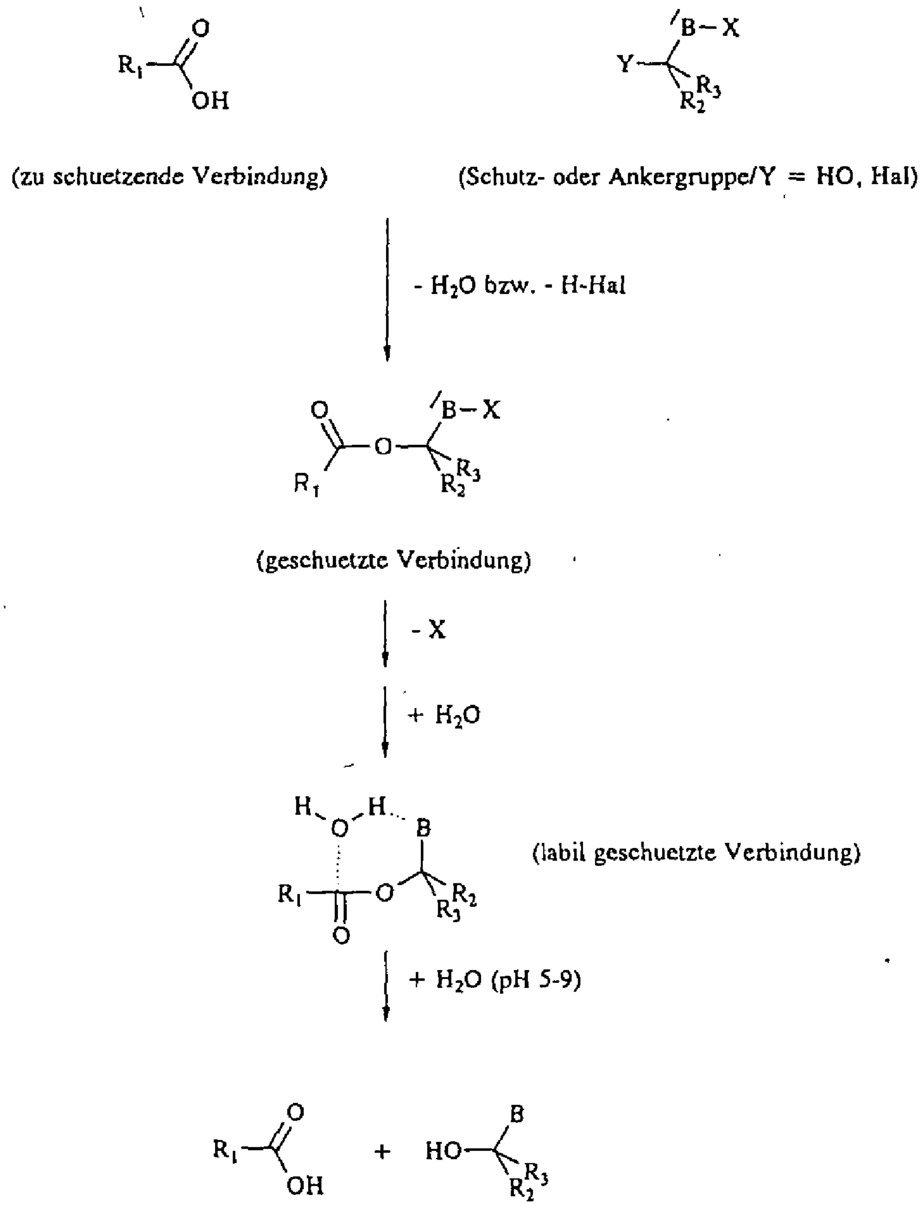


Erfindungsgemäß kann B ein aromatischer stickstoffhaltiger Fünfring und/oder X eine elektronenziehende Schutzgruppe sein, die die Basizität der basischen Gruppe B stark herabsetzt, und/oder
$R_2$ = H, Alkyl oder Aryl, jeweils ohne reaktive Funktion, und/oder
$R_3$ = H, Alkyl oder Aryl, jeweils ohne reaktive Funktion; HOOC, wodurch eine Ankergruppe vorgesehen werden kann; oder Alkyl oder Aryl, jeweils durch eine reaktive Funktion substituiert, die HO, $H_2N$, HS oder HOOC sein kann, wodurch eine Ankergruppe vorgesehen werden kann.

Beispielsweise kann B gegebenenfalls im Ring methylsubstituiertes Imidazolyl-yl oder Imidazolyl-ylmethylen, X eine Urethan- oder Alkoxycarbonyl-Schutzgruppe, $R_2$ = $C_{1-12}$-Alkyl oder Phenyl und/oder
$R_3$ = $C_{1-12}$-Alkyl, Phenyl, HOOC-$C_{1-12}$-alkyl, HOOC-phenyl, HO-$C_{1-12}$-alkyl, HO-phenyl, $H_2N$-$C_{1-12}$-alkyl, $H_2N$-phenyl, HS-$C_{1-12}$alkyl oder HS-phenyl.

Vorzugsweise ist B Imidazol-4-yl-methylen, Imidazol-4-yl, Imidazol-2-yl oder 5-Methylimidazol-4-yl und/oder X tert-Butoxycarbonyl (Boc).

Gemäß einer weiteren Ausführungsform betrifft die Erfindung Ester von Säuren, die durch Veresterung mit einer erfindungsgemäßen Verbindung gewinnbar sind. Bei derartigen Säuren kann es sich um Mono-, Di- oder Oligopeptide und deren Derivate handeln.

Gemäß einer weiteren Ausführungsform betrifft die Erfindung schließlich die Verwendung erfindungsgemäßer Verbindungen und Ester bei der Peptidsynthese.

Experimenteller Teil

Alllgemeiner experimenteller Teil : Es wurden folgende analytisch-spektroskopische Apparaturen verwendet.- $^{1}H$-NMR/$^{13}C$-NMR : Bruker Modell AM-300 und WM-400 mit Tetrametylsilan (TMS) als interner Standard.- Signalmultiplizität : s = Singulett, d = dublett, t = Triplett, q = Quartett, $^{n}J_{H,H}$ = magnetische Kopplung über n Bindungen zwischen benachbarten Protonen.- FAB-MS : Kratos MS 50 TC RF mit neutralen Xenonstrahl (8-9 kV) und Finnigan Mat, Mass SWpectrometer 8430 mit 3-Nitrobenzylalkohol als Matrix. Die Proben wurden in DMSO vorgelegt. UV/VIS : Carl Zeiss Modell PMQ II in Quarzküvetten mit 10 mm optischer Länge. ε(Dibenzofulven-Piperidin-Addukt/MeOH) = 5570. RP-$C_{18}$-HPLC : Analyt. IIPLC : Pharmacia/LKB Pump P 3500, Liquid Chromatographie Controller LCC 500 Plus bzw. LKB 2249 Gradient Pump, LKB 2141 Variable Wavelength Monitor, Dreikanalflachbettschreiber auf Machery-Nagel Nucleosil 300-7 $C_{18}$ 250x4.

Spezieller experimenteller Teil :

2-1H-(5-Methyl-imidazol-4-yl)-2-hydroxyessigsäure **(1)** : ($C_6H_8N_2O_3$) 18.25 g (0.17 mol) 4-Methyl-5-1H-imidazolylcarbaldehyd [hergestellt nach Literaturvorschrift: Papadopoulos, E.P., Jarrar, A.und Issidorides, C.H., J. Chem. Soc. Chem. Comm. 615 (1966)]werden in 80 ml Wasser gelöst und bei 0°C unter Rühren 23.65 g (0.36 mol) Kaliumcyanid, 30 ml konz. Salzsäure (37 %) und 66 ml Acetanhydrid zugesetzt. Es wird 1 h bei 0°C gerührt und weitere 24 h bei RT. Es werden bei RT 15 ml konz. Salzsäure und 66 ml Acetanhydrid zugesetzt und weitere 24 h bei RT gerührt. Nach Zusetzen von 250 ml Wasser und 250 ml konz. Salzsäure wird unter Rückfluß auf 100°C erwärmt. Nach 2 h werden weitere 250 ml konz. Salzsäure zugegeben und 12 h bei 80°C gerührt. Die Lösung wird eingeengt und entweder durch präp. HPLC-Chromatographie auf Latek RP-$C_{18}$ HL 10μ 300x40 aufgereinigt (Ausb. 95 % d. Th.) oder (**1**) nach, über das Silbersalz gereinigt [Stewart, C.P., J. Med. Chem. 130 (1923)]. Der Rückstand wird in Wasser gelöst und in salpetersaurer Lösung mit Sibernitrat gefällt. Nach Filtration der Lösung wird Bariumhydroxid zugesetzt und das Silbersalz von (**1**) zusammen mit überschüssigen Siberoxid abfiltriert. Das Filtrat wird in Wasser suspendiert und Schwefelwasserstoff eingeleitet. Nach Filtration der Lösung wird überschüssiges Barium durch vorsichtige Zugabe von Schwefelsäure gefällt. Nach erneuter Filtration wird die Lösung eingeengt und (**1**) durch mehrfache fraktionierende Kristallisation aus wässrigen Ethanol gewonnen.- Ausb. 42 % d. Th.

$R_2$ : H
$R_3$ : COOH
B : 5-Methyl-imidazol-4-yl-
X : H

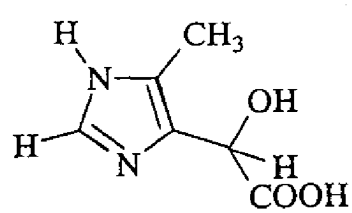

$^{1}H$-NMR (400 MHz, $D_2O$) : δ = 8.57 (s, 1H, 2-H), 5.59 (s, 1H, CHOH), 2.33 (s, 3H, $CH_3$).-$^{13}C$-NMR (75 MHz, $D_2O$) : δ = 174.0 (s, COOH), 135.2 (d, C-2), 132.3 (s, C-5), 119.9 (d, C-4), 65.5 (d, CHOH), 9.5 (q, $CH_3$).- MS (FAB) : M/Z (3-NBA) = 157 ($[M+H]^{\oplus}$).

2-1H-(Imidazol-4-yl)-2-hydroxyessigsäure (**2**) : ($C_5H_6N_2O_3$) Die Synthese erfolgte analog zu (**1**) ausgehend von 4-1H-Imidazolcarbaldehyd[hergestellt nach Literaturvorschrift: Weidenhagen, R. und Herrmann, R., Chem. Ber. 1955 (1953)] Ausb. 92 % d. Th. (RP-$C_{18}$-Chromatographie/Eluent : Wasser / 0.1 %TFA) oder wie unter (**1**) durch fraktionierende Kristallisation aus Wasser/Ethanol.

$R_2$ : H
$R_3$ : COOH
B : Imidazol-4-yl-
X : H

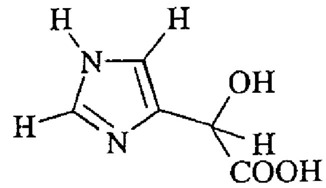

$^1$H-NMR (400 MHz, $D_2O$) : δ = 8.70 (s, 1H, 2-H), 7.51 (s, 1H,4-H), 5.54 (s, 1H, CHOH).-$^{13}$C-NMR (75 MHz, $D_2O$) : δ = 173.5 (s, COOH), 135.0 (d, C-2), 131.2 (s, C-5), 117.9 (d, C-4), 65.2 (d, CHOH) - MS (FAB) : M/Z (3-NBA) = 143 ([M+H]$^{\oplus}$).

2-1H-(Imidazol-2-yl)-2-hydroxyessigsäure (**3**) : ($C_5H_6N_2O_3$) Die Synthese erfolgte analog zu (**1**) ausgehend von 2-1H-Imidazolcarbaldehyd [kommerziell erhältlich, z.B. Aldrich]. Ausb. 82 % d. Th. (RP-$C_{18}$-Chromatographie/Eluent Wasser / 0.1 %TFA).

$R_2$ : H
$R_3$ : COOH
B : Imidazol-2-yl-
X : H

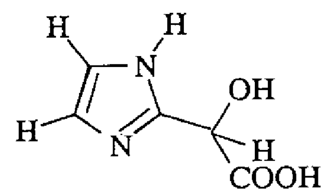

$^1$H-NMR (400 MHz, $D_2O$) : δ = 7.38 (s, 2H, H-1), 5.69 (1H, s, H-2).- $^{13}$C-NMR (75 MHz, $D_2O$): δ = 171.1 (s, COOH), 144.0 (s, C-2), 120.1 (d, C-4/C-5), 66.2 (d, CHOH).- MS (FAB) : M/Z (3-NBA) = 143 ([M+H]$^{\oplus}$).

3-1H-(N$^{im}$-tert-Butoxycarbonyl-imidazol-4-yl)-2-hydroxy-propionsäure (**4**) : ($C_{11}H_{16}N_2O_5$) 248 mg 3-1H-Imidazol-2-hydroxy-propionsäure (1.6 mmol) [kommerziell erhältlich, z.B. Sigma] werden unter Zugabe von 223 µl (1.6 mmol) in 2.5 ml abs. DMF unter Rühren bei 4°C suspendiert und 344 µl (2.4 mmol) tert-Butoxycarbonylazid zugegeben. Es wird 2 d bei 4°C gerührt und die Lösung bei 40°C am Rotationsverdampfer vollständig eingeengt. Der Rückstand wird in abs. Dioxan gelöst und der entstehende kristalline Niederschlag abfiltriert. Lyophilisation im HV ergibt einen farblosen, zähen Sirup. 356 mg (1.4 mmol), Ausb. 87 % d. Th.

$R_2$ : H
$R_3$ : COOH
B : Imidazol-4-ylmethyl-
X : tert-Butoxycarbonyl- (Boc)

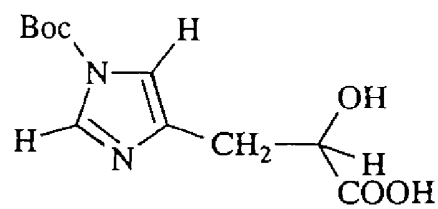

$^1$H-NMR (300 MHz, $CDCl_3$) : δ = 8.15 (d, 1H, 2-H, $^4J_{H,H}$=1.25 Hz), 7.27 (s, 1H, 4-H), 4.39 (t, 1H, CHOH, $^3J_{H,H}$=4.5 Hz), 3.14 (AB-dd, 1H, $-CH_2-$, $^2J_{H,H}$=14.8 Hz, $^3J_{H,H}$=4.0 Hz), 3.02 (AB-dd, 1H, $-CH_2-$, $^2J_{H,H}$=14.8 Hz, $^3J_{H,H}$=5.0 Hz), 1.57 (s, 9H, $CH_3$).- $^{13}$C-NMR (75 MHz, $CDCl_3$) : δ = 176.1 (s,COOH), 146.3 (s, N-COO-), 136.8 (d, C-2), 135.9 (s, C-5), 116.1 (d, C-4), 86.4 (s, $C(CH_3)_3$), 68.5 (d, CHOH), 67.0 (t, $-CH_2-$), 27.8 (q, $CH_3$).- MS (FAB, DCHA-Derivat) : M/Z (3-NBA) = 295 ([M+K]$^{\oplus}$), 279 ([M+Na]$^{\oplus}$), 257 ([M+H]$^{\oplus}$), 201 ([M-57]$^{\oplus}$,-tBu), 182 ([DCHA+H]$^{\oplus}$).

2-1H-(N$^{im}$-tert-Butoxycarbonyl-imidazol-4-yl)-2-hydroxy-essigsäure (**5**) : ($C_{10}H_{14}N_2O_5$) Die Synthese erfolgte analog zu (**4**) ausgehend von (**2**). Ausb, 52 % d. Th.

$R_2$ : H
$R_3$ : COOH
B : Imidazol-4-yl-
X : tert-Butoxycarbonyl- (Boc)

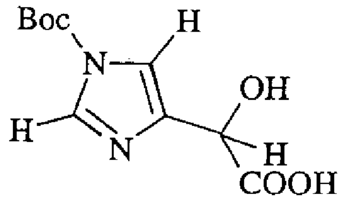

$^1$H-NMR (400 MHz, $CDCl_3$) : δ = 8.15 (s, 1H, 2-H), 7.27 (s, 1H, 4-H), 5.14 (s, 1H, CHOH), 1.58 (s, 9H, $CH_3$).-$^{13}$C-NMR (75 MHz, $CDCl_3$) : δ = 176.1 (s,COOH), 146.3 (s, N-COO-), 136.8 (d, C-2), 135.9 (s, C-5), 116.1 (d, C-4), 86.4 (s, $C(CH_3)_3$), 66.5 (d, CHOH), 27.8 (q, $CH_3$).

2-1H-($N^{im}$-tert-Butoxycarbonyl-5-methyl-imidazol-4-yl)-2-hydroxy-essigsäure (**6**) : ($C_{11}H_{16}N_2O_5$) Die Synthese erfolgte analog zu (**4**) ausgehend von (**1**) durch Umsetzung mit Pyrokohlensäure-di-tert-butylester oder tert-Butoxycabonylazid in DMF. 273 mg (1.1 mmol) Ausb. 67 % d. Th. (weißes Lyophilisat aus Dioxan).

$R_2$ : H
$R_3$ : COOH
B : 5-Methyl-imidazol-4-yl-
X : tert-Butoxycarbonyl- (Boc)

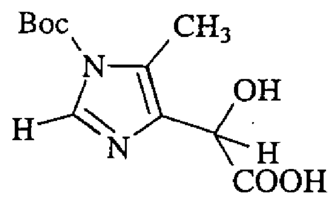

$^1$H-NMR (400 MHz, $CDCl_3$) δ = 8.15 (s, 1H, 2-H), 5.14 (s, 1H, CHOH), 2.46 (s, 3H, $CH_3$), 1.58 (s, 9H, $CH_3$).-$^{13}$C-NMR (75 MHz, $CDCl_3$): δ = 174.6 (s, COOH), 147.1 (s, N-COO-), 137.0 (d, C-2), 136.2 (s, C-5), 127.2 (s, C-4), 86.4 (s, $C(CH_3)_3$), 66.5 (d, CHOH). 27.8 (q, $CH_3$).- MS (FAB) : M/Z (3-NBA) = 279 ([M+Na]$^{\oplus}$), 257 ([M+H]$^{\oplus}$), 201 ([M-57+H]$^{\oplus}$, tBu).

2-1H-($N^{im}$-tert-Butoxycarbonyl-imidazol-2-yl)-2-hydroxy-essigsäure (**7**) : ($C_{10}H_{14}N_2O_5$) Die Synthese erfolgte analog zu (**4**) ausgehend von (**3**) durch Umsetzung mit Pyrokohlensäure-di-tert-butylester in DMF. Ausb. 51 % d. Th.- $^1$H-NMR (400 MHz, $CDCl_3$): δ = 8.62 (s, 1H, 4-H), 7.27 (s, 1H, 5-H), 5.56 (s, 1H, CHOH), 1.58 (s, 9H, $CH_3$).

$R_2$ : H
$R_3$ : COOH
B : Imidazol-2-yl-
X : tert-Butoxycarbonyl- (Boc)

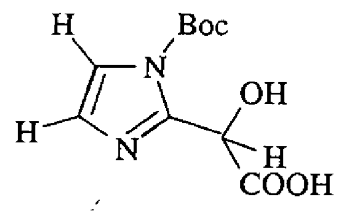

3-1H-(Imidazol-4-yl)-2-(phenylalanyl-phenylalanyl-glycyloxy)-propionyl-β-alanin (**8a**): ($C_{29}H_{34}N_6O_7$) MS (FAB) : M/Z (3-NBA) = 712 ([M+Cs+H]$^{\oplus}$), 579 ([M+H]$^{\oplus}$).

$R_1$ : H-Phe-Phe-Gly-O-
$R_2$ : H
$R_3$ : CONH-$CH_2$-$CH_2$-COOH
B : Imidazol-4-ylmethyl-
X : H

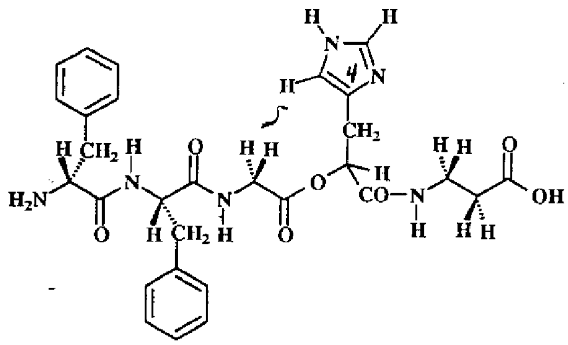

[2-1H-(5-Methyl-imidazol-4-yl)-2-(phenylalanyl-phenylalanyl-glycyloxy)]-acetyl-β-alanin(**9a**): ($C_{29}H_{34}N_6O_7$) MS (FAB) : M/Z (3-NBA) = 712 ($[M+Cs+H]^{\oplus}$), 579 ($[M+H]^{\oplus}$).

$R_1$ : H-Phe-Phe-Gly-O-
$R_2$ : H
$R_3$ : CONH-$CH_2$-$CH_2$-COOH
B : 5-Methyl-4-1H-imidazolyl-
X : H

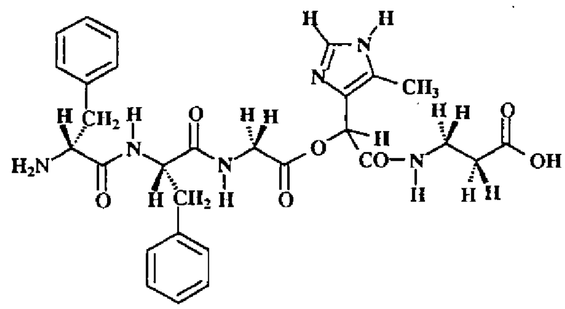

[2-1H-Imidazol-4-yl-2-(phenylalanyl-phenylalanyl-glycyloxy)]-acetyl-β-alaninyl-carrier(**10b**):

$R_1$ : H-Phe-Phe-Gly-O-
$R_2$ : H
$R_3$ : CONH-$CH_2$-$CH_2$-COOH
B : 5-Methyl-imidazol-4-yl-
X : H

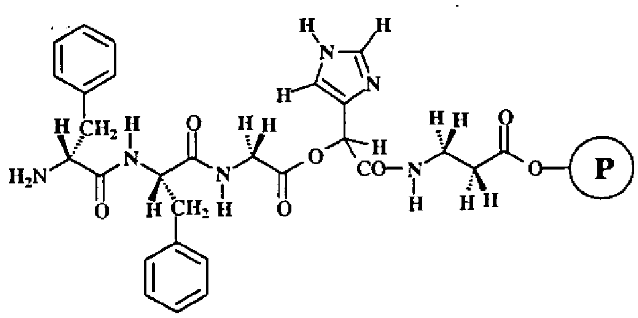

[Phenylalanyl-phenylalanyl-giycyloxy)]-acetyl-β-alanin (**11a**) :

$R_1$ : H-Phe-Phe-Gly-O-
$R_2$ : H
$R_3$ : CONH-$CH_2$-$CH_2$-COOH
B : H

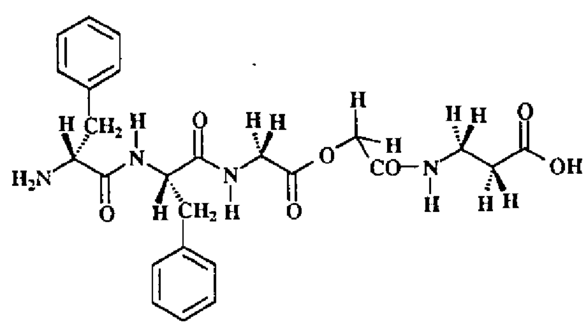

Das Schutzgruppenkonzept wurde am Beispiel der oben aufgeführten Verbindungen mit B = 1H-Imidazolyl

und X = tert-Butyloxycarbonyl sowohl in Lösung als auch gebunden an einen festen Papierträger (Whatman 3MM) mit Hinblick auf die spezielle Verwendung als orthogonale Ankergruppe für die Festphasenpeptidsynthese überprüft.

Test in Lösung

Die Verbindungen (**8**) und (**9**) wurden an einen p-Benzyloxybenzyl-derivatisierten PolystyrolHarz ('Wang-Harz' der Firma Novabiochem) synthetisiert. Dazu wurde $N^{\beta}$-9-Fluorenylmethoxycarbonyl-β-alanin an das Harz durch Aktivierung der Carboxylfunktion mit Mesitylensulfonyl-3-nitro-1.2.4-triazol (MSNT) unter Katalyse von N-Methylimidazol (MeIm) in DCM verestert [Blankemeyer-Menge, B. und Frank, R., Tetrahedron Lett, 31, 1701 (1990)]. Nach Abspaltung der Aminoschutzgruppe mit einem Überschuß an 20% Piperidin in DMF wurde (**4**) bzw. (**6**) durch N.N-Diisopropylcarbodiimid (DIC) (1.2 eq.) / Hydroxybenzotriazol (HOBt) (2.0 eq.)-Aktivierung der Carboxyl-Funktion in DMF an die gebundene H-βAlanin-Einheit gekoppelt. Die schrittweise Synthese des Tripeptides H-Phe-Phe-Gly- begann mit der MSNT/MeIm-aktivierten Veresterung des $N^{\alpha}$-Fmoc-Gly-OH an die Ankergruppe. Im weiteren wurde nach üblichen Peptidsynthesebedingungen unter Aktivierung von DIC/HOBt nach der $N^{\alpha}$-Frnoc/tBu-Strategie verfahren [Fields, G. B. and Noble, R.L., Int. J. Peptide Protein Res. 35, 161-214 (1990)]. Alle Kopplungsschritte verliefen unter nahezu quantitativer Ausbeute (UV/VIS-Analytik des Dibenzofulven/Piperidin-Adduktes). Die Verbindungen (**8a**) und (**9a**) wurden vom festen Träger mit Trifluoressigsäure (TFA)/ Dichlormethan(DCM) (1:1) (2.5 % Triisobutylsilan (TIBS), 2.5 % Wasser) abgespalten und RP-$C_{18}$-chromatographisch aufgereinigt. Dabei wird gleichzeitig die Imidazolylschutzgruppe X = Boc abgespalten. Als direkten Vergleich zu den hier speziell beschriebenen Ankergruppen wurde die Verbindung H-Phe-Phe-Gly-O-Glycolsäure-βAla-OH (**11a**) nach dem oben beschriebenen Verfahren an fester Phase synthetisiert, wie oben abgespalten und durch RP-$C_{18}$-HPLC aufgereinigt.
Die am Imidazolyl-Rest (B) entschützten, jetzt labil geschützten Verbindungen (**8a**), (**9a**) sowie die Vergleichssubstanz (**11a**) wurden in Lösung auf die Labilität der Esterbindung gegenüber den unten aufgeführten wässrigen Puffern untersucht. Tabelle 1 gibt die Ergebnisse wieder.

Verwendete Puffer :

(a) $NaH_2PO_4/Na_4HPO_4$/0.1M/pH 7.0/$H_2O$
(b) $NaH_2PO_4/Na_2HPO_4$/0.1M/pH 7.5/$H_2O$
(c) $NaH_2PO_4/Na_2HPO_4$/0.01M/pH 7.0/$H_2O$
(d) $NaH_2PO_4/Na_2HPO_4$/0.01M/pH 7.5/$H_2O$
(e) $Na_3BO_4/H_3BO_4$/0.05 M/pH 7.6/$H_2O$
(f) $Na_3BO_4/H_3BO_4$/0.05 M/pH 8.0/$H_2O$
(g) tris-Hydroxymethylaminomethan-hydrochlorid (Tris.HCl)/0.01 M/pH 7.6/$H_2O$
(h) tris-Hydroxymethylaminomethan-hydrochlorid (Tris.HCl)/0.01 M/pH 8.0/$H_2O$
(i) $NaHCO_3/Na_2CO_3$/0.05 M/pH 9.6 /$H_2O$
(j) $NaHCO_3/Na_2CO_3$/0.05 M/pH 10.0 /$H_2O$
(k) DMF/Wasser = 1:9 (pH 7.0)
(1) Triethylammoniumacetat (TEAAc)/0.01 M/pH 7.0/$H_2O$

| min | (c) $NaH_2PO_4/Na_2HPO_4$/0.01M/pH 7.0/$H_2O$ (25°C) | | | | | | (c) $NaH_2PO_4/Na_2HPO_4$/0.01M/pH 7.0/$H_2O$ (50°C) | | | | | | |
|---|---|---|---|---|---|---|---|---|---|---|---|---|---|
| | 120 | 240 | 510 | 600 | 720 | 840 | 10 | 15 | 30 | 45 | 120 | 270 | 840 |
| (8) | (-) | 33 | 55 | (-) | 100 | 100 | (-) | (-) | (-) | 46 | (-) | 100 | 100 |
| (9) | 45 | (-) | (-) | 100 | 100 | 100 | 100 | 100 | 100 | 100 | 100 | 100 | 100 |
| (11) | (-) | (-) | (-) | (-) | (-) | 5 | (-) | (-) | (-) | (-) | 20 | (-) | (-) |

| min | (d) $NaH_2PO_4/Na_2HPO_4$/0.01M/pH 7.5/$H_2O$ (25°C) | | | | | | (d) $NaH_2PO_4/Na_2HPO_4$/0.01M/pH 7.5/$H_2O$ (50°C) | | | | | | |
|---|---|---|---|---|---|---|---|---|---|---|---|---|---|
| | 45 | 150 | 600 | 720 | 780 | 840 | 5 | 30 | 45 | 60 | 120 | 270 | 510 |
| (8) | (-) | (-) | (-) | (-) | (-) | (-) | (-) | (-) | 46 | (-) | (-) | 100 | 100 |
| (9) | 45 | 66 | 100 | 100 | 100 | 100 | 5 | (-) | (-) | 100 | 100 | 100 | 100 |
| (11) | (-) | (-) | (-) | (-) | (-) | 5 | (-) | 0 | (-) | (-) | 22 | (-) | (-) |

| min | (j) $NaHCO_3/Na_2CO_3$/0.05 M/pH 10.0 (25°C) | | | | | | Tris / 0.01 M /$H_2O$ | | |
|---|---|---|---|---|---|---|---|---|---|
| | 2.5 | 5 | 30 | 45 | 510 | 840 | g/720/50°C | g/270/25°C | g/45/25°C |
| (8) | 100 | 100 | 100 | 100 | 100 | 100 | (-) | 50 | 48 |
| (9) | (-) | 100 | 100 | 100 | 100 | 100 | 100 | (-) | (-) |
| (11) | (-) | (-) | (-) | 49* | (-) | (-) | (-) | (-) | (-) |

Tabelle 1 : Reaktionsfortschritt der Esterhydrolyse [%] von (8) und (9) im Vergleich zu (11) in verschiedenen Puffern bei 25°C und 50 °C; (-) = nicht bestimmt, * = 50°C.

Test am polymeren Träger

Die analogen, polymergebundenen Verbindungen zu (**8a**), (**9a**) und (**11a**) wurden wie oben aber auf Papierrundfiltem (Whatman 3MM synthetisiert). Die Behandlung mit TFA/DCM (2.5 % TIBS, 2.5 % $H_2O$) spaltet in diesem speziellen Fall nur die Imidazolylschutz-gruppe X = Boc ab, berührt jedoch die Esterbindung zwischen C-terminalen Carboxylfunktion und dem festen Träger nicht (polymergebundene Verbindungen werden im weiteren mit einem b gekennzeichnet). Zusätzlich zu (**8b**), (**9b**) und (**11b**) wurde eine an die feste Phase gebundene Verbindung (**10b**) mit dem Ankermolekül (**5**) synthetisiert. Nach Abspalten der Schutzgruppe X vom polymergebundenen (**8b**)-(**10b**) wird der Filter 2 x 5 min mit dem Abspaltreagenz, 3 x 10 min mit 1 % HCl in Methanol/Wasser (1:1) und 3 x 10 min mit 1 M Essigsäure/Wasser gewaschen und im HV 12 h getrocknet. In den Waschlösungen ist kein Tripeptid H-Phe-Phe-Gly-OH nachzuweisen.

Die am Imidazolyl-Rest entschützten Verbindungen (**8a**), (**9a**) sowie die Vergleichssubstanz (**11a**) wurden in Lösung auf die Labilität der Esterbindung gegenüber den unten aufgeführten wässrigen Puffern untersucht. Tabelle 1 gibt die Ergebnisse wieder.

Tabelle 2 :

| Abspaltung des Tripeptides H-Phe-Phe-Gly-OH von den festphasengebundenen Verbindungen (**8b**)-(**11b**) in verschieden Puffern bei 50°C über 12 h. Die Meßfehler sind mit einem Fehler von ca. 20 % behaftet. | | | | | | | | | |
|---|---|---|---|---|---|---|---|---|---|
| | (a) | (b) | (c) | (d) | (e) | (f) | (h) | (k) | (1) |
| (**8b**) | 100 | 100 | 100 | 100 | 100 | 100 | 80 | 5 | 90 |
| (**9b**) | 100 | 100 | 100 | 100 | 100 | 100 | 90 | 5 | 60 |
| (**10b**) | 100 | 100 | 100 | 100 | 100 | 100 | 50 | 5 | 80 |
| (**11b**) | nd | 25 | nd | 25 | 20 | 40 | nd | 1 | 25 |

Die Ergebnisse zeigen deutlich,

- daß die mit X geschützte Schutzgruppe unter den basischen Reaktionsbedingungen (z.B. 20 % Piperidin in DMF) der Synthese von $R_1$ stabil ist
- daß die Esterbindung der Schutzgruppe an die Carboxylverbindung unter sauren wäßrigen Bedingungen stabil ist und die entsprechend geschützten Verbindungen gereinigt werden können
- daß die basische Funktion des Typs von Schutz- bzw. Ankergruppe zu einer drastisch erhöhten Hydrolyserate im Vergleich zu den nicht substituierten Glycolsäurederivaten führt.
- daß eine Abspaltung der Schutzgruppe (ohne X) auch unter neutralen Reaktionsbedingungen (pH = 7) möglich ist.

3-1H-($N^{im}$-tert-Butoxycarbonyl-imidazol-4-yl)-2-bromo-propionsäure **(12)** : ($C_{11}H_{15}BrN_2O_4$) Die Synthese erfolgte analog zu (**4**) ausgehend von 3-1H-Imidazol-4-yl-2-bromo-propionsäure (z.B. kommerziell erhältlich bei Sigma). Ausb. 67 % d. Th.

$R_2$ : H
$R_3$ : COOH
B : Imidazol-4-yl-
X : tert-Butoxycarbonyl- (Boc)

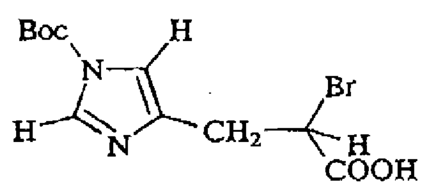

und Hydroxylfunktion (-OH) ersetzt durch Brom-Funktion (-Br)
$^1$H-NMR (400 MHz, $CDCl_3$) : δ = 8.15 (s, 1H, 2-H), 7.27 (s, 1H, 4-H), 5.55 (s, 1H, C<u>H</u>Br), 1.58 (s, 9H, $CH_3$).- $^{13}$C-NMR (75 MHz, $CDCl_3$) : = δ = 176.1 (s,COOH), 146.3 (s, N-COO-), 136.8 (d, C-2), 135.9 (s, C-5), 116.1 (d, C-4). 86.4 (s, <u>C</u>$(CH_3)_3$), 73.5 (d, CHOH), 27.8 (q, $CH_3$).

Verwendete Abkürzungen :

Aminosäurederivate nach IUPAC-IUB [J. Biol. Chem. 260, 14(1983)

| | |
|---|---|
| Boc | tert.-Butyloxycarbonyl |
| DCHA | Dicyclohecylammonium |
| DCM | Dichlormethan |

| | |
|---|---|
| DIC | N,N'-Diisopropylcarbodiimid |
| DMF | Dimethylformamid |
| DMSO | Dimethylsulfoxid |
| FAB-MS | "Fast Atom Bombardement" Massenspektroskopie |
| Fmoc | 9-Fluorenylmethoxycarbonyl |
| Hal | Halogen |
| HOBt | N-Hydroxybenzotriazol |
| HPLC | Hochdruckflüssigchromatographie |
| HV | Hochvakuum |
| MeIm | N-Methylimidazol |
| MSNT | Mesitylensulfonyl-3-vitro-12.4-triazol |
| 3-NBA | 3-Nitrobenzylalkohol |
| NMR | Kernresonanzspektroskopie |
| TIBS | Triisobutylsilan |
| TFA | Trifluoressigsäure |

1. Verbindung der Formel

$$Y\text{-}C(R_2)(R_3)\text{-}B\text{-}X$$

wobei die Verbindung mit einer Carboxyl-Funktion durch Substituieren der Y-Gruppe unter Ausbildung einer Esterbindung umgesetzt werden kann, wodurch eine Schutzgruppe und insbesondere eine Ankergruppe für die Carboxyl-Funktion vorgesehen wird, wobei
Y = HO, Cl, Br oder I,
B = basische Gruppe, die durch X geschützt ist, hydrolyseempfindliche Acylverbindungen eingeht und entschützt intramolekular die Hydrolyse der Esterbindung katalysieren kann,
X = eine Schutzgruppe für die basische Gruppe B bedeutet und deren Katalysevermögen blockiert,
$R_2$ und $R_3$ = beliebige Reste sind, die nicht die Bildung der Esterbindung und deren Hydrolyse beeinträchtigen.

2. Verbindung nach Anspruch 1 mit
B = aromatischer stickstoffhaltiger Fünfring und/oder
X = elektronenziehende Schutzgruppe, die die Basizität der basischen Gruppe B stark herabsetzt, und/oder
$R_2$ = H, Alkyl oder Aryl, jeweils ohne reaktive Funktion, und/oder
$R_3$ = H, Alkyl oder Aryl, jeweils ohne reaktive Funktion; HOOC, wodurch eine Ankergruppe vorgesehen werden kann; oder Alkyl oder Arly, jeweils durch eine reaktive Funktion substituiert, die HO, $H_2N$, HS oder HOOC sein kann, wodurch eine Ankergruppe vorgesehen werden kann.

3. Verbindung nach Anspruch 1 oder 2 mit
B = gegebenenfalls im Ring methylsubstituiertes Imidazol-yl oder Imidazol-yl-methylen,
X = Urethan- oder Alkoxycarbonyl-Schutzgruppe,
$R_2$ = $C_{1-12}$-Alkyl oder Phenyl und/oder
$R_3$ = $C_{1-12}$-Alkyl, Phenyl, HOOC-$C_{1-12}$-alkyl, HOOC-phenyl, HO-$C_{1-12}$-alkyl, HO-phenyl, $H_2N$-$C_{1-12}$-alkyl, $H_2N$-phenyl, HS-$C_{1-12}$-alkyl oder HS-phenyl.

4. Verbindung nach einem der vorhergehenden Ansprüche mit
B = Imidazol-4-yl-methylen, Imidazol-4-yl, Imidazol-2-yl oder 5-Methylimidazol-4-yl und/oder
X = tert-Butoxycarbonyl (Boc).

5. Ester einer Säure, gewinnbar durch Veresterung mit einer Verbindung gemäß einem der vorhergehenden Ansprüche.

6. Verwendung einer Verbindung oder eines Esters gemäß einem der vorhergehenden Ansprüche bei der Peptidsynthese.

**Patentansprüche**

1. Synthese eines Naturstoffs mit carboxyl-Fünktion, bei der man

(i) eine Verbindung der Formel

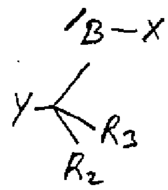

wobei
Y = HO, Cl, Br oder I,
B = basische Gruppe,
X = Schutzgruppe für B, die die Basizität von B soweit herabsetzt, daß die intramolekulare Hydrolyse einer Esterbindung erst nach Abspaltung von X erfolgen kann,
$R_2$ = H, Alkyl oder Aryl, und
$R_3$ = HOOC; oder Alkyl oder Aryl, jeweils durch eine reaktive Funktion substituiert, die eine Ankergruppe bildet,
mit einem Trägermaterial verknüpft,

(ii) danach die mit dem Träger verknüpfte Verbindung mit einer Carboxyl-Funktion (COOH) einer Verbindung der Formel

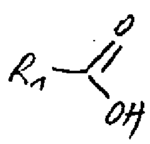

mit $R_1$ = Rest der Verbindung mit Carboxyl-Funktion unter Ausbildung einer Esterbindung umsetzt und einen Ester synthetisiert,

(iii) danach die Schutzgruppe X selektiv abspaltet,

(iv) danach das angefallene Zwischenprodukt reinigt und

(v) danach das angefallene Zwischenprodukt in wässeriger Lösung zerfallen lässt und die Carbonsäure als Syntheseprodukt mit freier Carboxyl-Funktion gewinnt.

2. Synthese nach Anspruch 1, bei der man

(i) eine Verbindung der Formel gemäß Anspruch 1 Stufe (i), wobei
Y = HO, Cl, Br oder I,
B = aromatischer stickstoffhaltiger Fünfring,
X = Schutzgruppe für B, die die Basizität von B soweit herabsetzt, daß die intramolekulare Hydrolyse der Esterbindung erst nach Abspaltung von X erfolgen kann,
$R_2$ = H, Alkyl oder Aryl, und
$R_3$ = HOOC; oder Alkyl oder Aryl, jeweils durch eine reaktive Funktion substituiert, die eine Ankergruppe bildet,
mit einem Trägermaterial verknüpft,

(ii) danach die verknüpfte Verbindung mit einer Carboxyl-Funktion (COOH) unter Ausbildung einer Esterbindung eines Esters eines Mono-, Di- oder Oligopeptids umsetzt und ein Peptid synthetisiert,

(iii) danach die Schutzgruppe X selektiv abspaltet,

(iv) danach das angefallene Zwischenprodukt reinigt und

(v) danach das angefallene Zwischenprodukt in wässeriger Lösung zerfallen lässt und das Peptid als Syntheseprodukt mit freier Carboxyl-Funktion gewinnt.

**3.** Synthese nach Anspruch 1 oder 2, **dadurch gekennzeichnet, dass** man Stufe (v) in physiologischer Pufferlösung durchführt.

**4.** Synthese nach einem der vorhergehenden Ansprüche, **dadurch gekennzeichnet, dass** man die Synthese bei Stufe (ii) unter basischen Reaktionsbedingungen durchführt.

**5.** Synthese nach einem der vorhergehenden Ansprüche, **dadurch gekennzeichnet, dass** man in der Verbindung der Formel gemäß Anspruch 1 $R_3$ durch $H_2N$, HS, OH oder COOH substituiert ist.

**6.** Synthese nach einem der vorhergehenden Ansprüche, **dadurch gekennzeichnet, dass** man eine Verbindung der Formel gemäß Anspruch 1 verwendet mit $R_3$ = HOOC-$C_{1-12}$-alkyl, HOOC-phenyl, HO-$C_{1-12}$-alkyl, HO-phenyl, $H_2N$-$C_{1-12}$-alkyl, $H_2N$-phenyl, HS-$C_{1-12}$alkyl oder HS-phenyl.

**7.** Synthese nach einem der vorhergehenden Ansprüche, **dadurch gekennzeichnet, daß** man eine Verbindung der Formel gemäß Anspruch 1 verwendet mit X = Urethan- oder Alkoxycarbonyl Schutzgruppe.

**8.** Synthese nach Anspruch 7, **dadurch gekennzeichnet, dass** man eine Verbindung der Formel gemäß Anspruch 1 verwendet mit
X = tert-Butoxycarbonyl (Boc).

**9.** Synthese nach einem der vorhergehenden Ansprüche, **dadurch gekennzeichnet, daß** man eine Verbindung der Formel gemäß Anspruch 1 verwendet mit $R_2$ = $C_{1-12}$-Alkyl oder Phenyl.

**10.** Synthese nach einem der Ansprüche 2 bis 9, **dadurch gekennzeichnet, daß** man eine Verbindung der Formel gemäß Anspruch 1 verwendet mit B = gegebenenfalls im Ring methylsubstituiertes Imidazol-yl oder gegebenenfalls im Ring methylsubstituiertes Imidazol-yl-methylen.

**11.** Synthese nach Anspruch 10, **dadurch gekennzeichnet, daß** man eine Verbindung der Formel gemäß Anspruch 1 verwendet mit
B = Imidazol-4-yl oder Imidazol-2-yl.

**12.** Synthese nach Anspruch 10, **dadurch gekennzeichnet, daß** man eine Verbindung der Formel gemäß Anspruch 1 verwendet mit
B = Imidazol-4-yl-methylen oder 5-Methyl-imidazol-4-yl.

A

Fig. 1

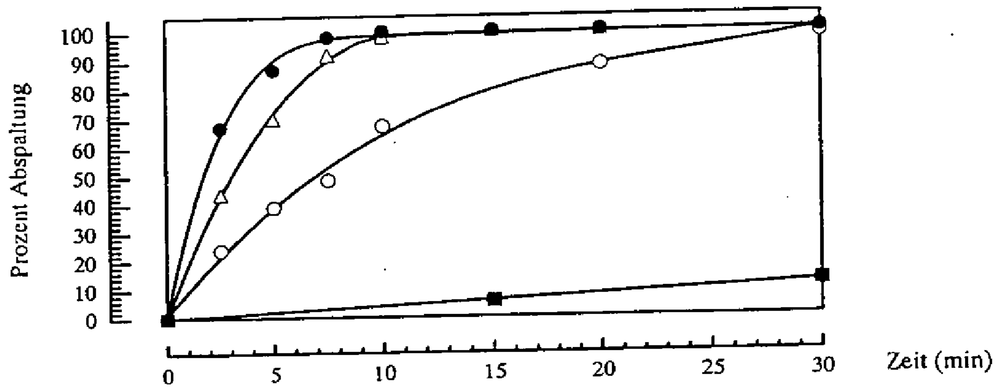


B

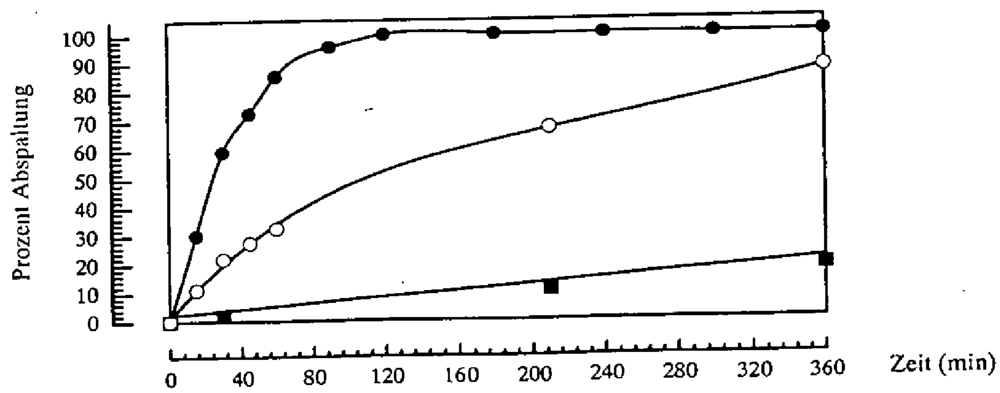


Kinetik der Peptidabspaltung von einigen Ankergruppen in Lösung. **A** : (•) Verbindung (**9a**) in 0.01 M $KH_2PO_4/NaHPO_4$ (pH 7.5), (△) Verbindung (**9a**) in 0.01 M $KH_2PO_4/Na_2HPO_4$ (pH 7.5), (○) Verbindung (**9a**) in 0.01 M TEAAc (pH 7.3), (■) Verbindung (**11a**) in 0.01 M $KH_2PO_4/Na_2HPO_4$ (pH 7.5) bei 50°C; B : (•) Verbindung (**9a**), (○) Verbindung (**8a**) und (■) Verbindung (**11a**) in 0.01 M $KH_2PO_4$/ $Na_2HPO_4$ (pH 7.5) bei 25°C.

Europäisches Patentamt

# EUROPÄISCHER RECHERCHENBERICHT

Nummer der Anmeldung
EP 02 02 7593

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
| A | WO 83 02448 A (RESEARCH CORP)<br>21. Juli 1983 (1983-07-21)<br>* Ansprüche * | 1-12 | C07D233/64<br>C07K1/06 |
| A | FR 1 472 013 A (SHIONOGI & CO)<br>10. März 1967 (1967-03-10)<br>* Seite 2; Ansprüche * | 1-12 | |
| A | EP 0 061 933 A (NAT RES DEV)<br>6. Oktober 1982 (1982-10-06)<br>* Ansprüche * | 1 | |
| A | EP 0 322 348 A (HOECHST AG)<br>28. Juni 1989 (1989-06-28)<br>* Ansprüche * | 1-12 | |
| D,P,A | PATEK M: "MULTISTEP DEPROTECTION FOR PEPTIDE CHEMISTRY"<br>INTERNATIONAL JOURNAL OF PEPTIDE AND PROTEIN RESEARCH, MUNKSGAARD, COPENHAGEN, DK,<br>Bd. 42, Nr. 2,<br>1. August 1993 (1993-08-01), Seiten 97-117, XP000464144<br>ISSN: 0367-8377<br>* das ganze Dokument * | 1-12 | RECHERCHIERTE SACHGEBIETE (Int.Cl.7)<br>C07D<br>C07K |
| P,A | WO 94 01409 A (FUJISAWA PHARMACEUTICAL CO ;KAGARA KOOJI (JP); KAWAI NOBUTAKA (JP))<br>20. Januar 1994 (1994-01-20)<br>* Ansprüche *<br>& EP 0 648 747 A<br>19. April 1995 (1995-04-19) | 1-12 | |

Der vorliegende Recherchenbericht wurde für alle Patentansprüche erstellt

| Recherchenort | Abschlußdatum der Recherche | Prüfer |
|---|---|---|
| DEN HAAG | 31. Januar 2003 | Chouly, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 02 02 7593

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

31-01-2003

| Im Recherchenbericht angeführtes Patentdokument | Datum der Veröffentlichung | Mitglied(er) der Patentfamilie | Datum der Veröffentlichung |
|---|---|---|---|
| WO 8302448 A | 21-07-1983 | US 4394519 A | 19-07-1983 |
| | | DE 3279468 D1 | 06-04-1989 |
| | | EP 0098865 A1 | 25-01-1984 |
| | | GR 77185 A1 | 11-09-1984 |
| | | IE 54589 B1 | 06-12-1989 |
| | | IT 1163037 B | 08-04-1987 |
| | | JP 58502207 T | 22-12-1983 |
| | | WO 8302448 A1 | 21-07-1983 |
| | | US 4508657 A | 02-04-1985 |
| | | US 4581167 A | 08-04-1986 |
| FR 1472013 A | 10-03-1967 | KEINE | |
| EP 0061933 A | 06-10-1982 | DE 3262745 D1 | 02-05-1985 |
| | | EP 0061933 A2 | 06-10-1982 |
| | | GB 2099816 A ,B | 15-12-1982 |
| | | JP 57183763 A | 12-11-1982 |
| | | US 4591648 A | 27-05-1986 |
| EP 0322348 A | 28-06-1989 | AT 101161 T | 15-02-1994 |
| | | AU 2743188 A | 22-06-1989 |
| | | DE 3842378 A1 | 06-07-1989 |
| | | DE 3887670 D1 | 17-03-1994 |
| | | DK 714588 A | 23-06-1989 |
| | | EP 0322348 A2 | 28-06-1989 |
| | | ES 2061714 T3 | 16-12-1994 |
| | | IE 62358 B1 | 25-01-1995 |
| | | IL 88741 A | 29-12-1994 |
| | | JP 2000243 A | 05-01-1990 |
| | | JP 2858766 B2 | 17-02-1999 |
| | | KR 128341 B1 | 03-04-1998 |
| | | PT 89297 A ,B | 29-12-1989 |
| | | US 5124478 A | 23-06-1992 |
| | | ZA 8809474 A | 30-08-1989 |
| WO 9401409 A | 20-01-1994 | JP 6016645 A | 25-01-1994 |
| | | JP 6172318 A | 21-06-1994 |
| | | CA 2139362 A1 | 20-01-1994 |
| | | CN 1089604 A | 20-07-1994 |
| | | EP 0648747 A1 | 19-04-1995 |
| | | HU 70049 A2 | 28-09-1995 |
| | | WO 9401409 A1 | 20-01-1994 |
| | | MX 9303933 A1 | 28-02-1994 |
| | | US 5523410 A | 04-06-1996 |

EPO FORM P0461

Für nähere Einzelheiten zu diesem Anhang : siehe Amtsblatt des Europäischen Patentamts, Nr.12/82